Europäisches Patentamt

European Patent Office

Office européen des brevets

(19)

(11) Veröffentlichungsnummer: **0 034 767**
**B1**

(12)

# EUROPÄISCHE PATENTSCHRIFT

(45) Veröffentlichungstag der Patentschrift:
21.12.83

(21) Anmeldenummer: 81100993.5

(22) Anmeldetag: 13.02.81

(51) Int. Cl.³: **C 07 C 29/15, C 07 C 1/24,**
**C 07 C 31/04, B 01 J 21/04,**
**B 01 J 23/02, B 01 J 23/76,**
**B 01 J 23/80, B 01 J 23/84,**
**B 01 J 23/86**

(54) **Verfahren zur Herstellung von Olefinen.**

(30) Priorität: 14.02.80 DE 3005550

(43) Veröffentlichungstag der Anmeldung:
02.09.81 Patentblatt 81/35

(45) Bekanntmachung des Hinweises auf die Patenterteilung:
21.12.83 Patentblatt 83/51

(84) Benannte Vertragsstaaten:
AT BE CH DE FR GB LI LU NL SE

(56) Entgegenhaltungen:
DE - A - 1 930 003
DE - A - 2 154 074
DE - A - 2 928 435
DE - B - 1 142 343
DE - B - 2 122 952
DE - B - 2 165 378
FR - A - 2 181 002
GB - A - 1 205 397

Patents Abstracts of Japan, Band 3, Nr. 79, 6. Juli 1979,
Seite 34C51
Patents Abstracts of Japan, Band 3, Nr. 50, 27. April
1979, Seite 36C44

(73) Patentinhaber: **SÜD-CHEMIE AG, Lenbachplatz 6,**
**D-8000 München 2 (DE)**

(72) Erfinder: **Hofstadt, Carl-Ernst, Dr. Dipl.-Chem.,**
**Wehrlestrasse 2, D-8000 München (DE)**
Erfinder: **Schneider, Michael, Dr. Dipl.-Chem.,**
**Waldparkstrasse 54a, D-8012 Ottobrunn-Riemerling (DE)**
Erfinder: **Kochloefl, Karel, Dr. Dipl.-Ing., Kreuzstrasse 2,**
**D-8052 Moosburg (DE)**

(74) Vertreter: **Patentanwälte Dipl.-Ing. Splanemann**
**Dipl.-Chem. Dr. B. Reitzner, Tal 13,**
**D-8000 München 2 (DE)**

## Verfahren zur Herstellung von Olefinen

Niedrige aliphatische Olefine, insbesondere Äthylen und Propen gehören heute zu den wichtigsten Grundstoffen der organischen Chemie. Beide Olefine werden heute in technischem Maßstab ausschließlich durch thermische Spaltung von gesättigten Kohlenwasserstoffen hergestellt, wobei in verschiedenen Ländern unterschiedliche Rohstoffe zur Verfügung stehen. So werden z. B. in Westeuropa mehr als 80% des Äthylens aus Naphtha, etwa 10 bis 15% durch Gasölspaltung und der Rest aus äthanreichem Erdgas gewonnen. Die Olefinproduktion ist völlig von eingeführten Erdöl abhängig. Um die Abhängigkeit der petrochemischen Industrie von der Erdöleinfuhr zu mildern, werden neue Wege zur Herstellung von Olefinen aus Synthesegas und Wasserstoff eingeschlagen.

Wie bekannt, kann Äthylen durch Hydrierung von Kohlenmonoxid mit Hilfe von Kobalt-, Nickel- oder Platin-Katalysatoren gemäß der DE-C-1 271 098 hergestellt werden. Jedoch ist die Wirtschaftlichkeit des Verfahrens durch die zu niedrige Äthylenausbeute beschränkt.

Einen weiteren Weg zur Synthese von Olefinen stellt die bekannte Fischer-Tropsch-Synthese dar. Weil bei der Anwendung von üblichen Eisenkatalysatoren die Olefine nur einen geringen Anteil des Produktspektrums bilden, wurden Eisenkatalysatoren mit $V_2O_5$ und ZnO oder MnO und ZnO (DE-A-2 518 964) oder nur mit MnO (DE-A-2 507 647) promotiert, um die Olefinbildung zu erhöhen. So erzeugt z. B. ein manganhaltiger Eisenkatalysator bei 265° C und einem Druck von 10,6 bar aus einem Synthesegas mit 54,5 Vol.-% CO und einer Raumgeschwindigkeit von 310 Liter Synthesegas pro Std. unter Liter Katalysator 8 g Äthylen und 28,2 g Propen/$Nm^3$. Außerdem werden 18,8 g Butene, 26,5 g Paraffine, 59,8 g flüssige Produkte und 198,3 g $CO_2$ pro $Nm^3$ Synthesegase, bei einem Gesamtumsatz an CO von 81% gebildet. Die Selektivität der Äthylen- und Propenbildung beträgt 11%. Die Wirtschaftlichkeit dieses Verfahrens wäre infolge der ziemlich niedrigen Selektivität nur durch die Möglichkeit der Ausnützung der anderen Produkte gegeben.

Der Erfindung liegt die Aufgabe zugrunde, ein Verfahren zur Herstellung von Olefinen, insbesondere von Äthylen und Propen, durch Dehydratisierung von aliphatischen Alkoholen zur Verfügung zu stellen, bei dem von Grundstoffen auf Kohlebasis ausgegangen werden kann.

Das erfindungsgemäße Verfahren ist dadurch gekennzeichnet, daß man

(a') aus löslichen Salzen des Kupfers, Zink und gegebenenfalls Aluminiums (Hauptbestandteile) durch gemeinsame Fällung mit Promotorverbindungen in alkalischem Medium einen Niederschlag erzeugt, der nach Entfernung der Fremdionen calciniert wird; oder

(b') ein Gemisch der Oxide des Kupfers, Zinks und gegebenenfalls des Aluminiums mit Lösungen der Promotorverbindungen imprägniert und das erhaltene Produkt calciniert, wobei die Promotorverbindungen Verbindungen von Chrom, Cer, Lanthan, Mangan oder von Kombinationen dieser Elemente darstellen, und wobei auf einer beliebigen Herstellungsstufe Alkaliverbindungen zugesetzt werden,

ein Methanol und höhere aliphatische Alkohole enthaltendes Alkoholgemisch erzeugt;

(b) aus diesem Alkoholgemisch das Methanol abtrennt;

(c) die höheren aliphatischen Alkohole, insbesondere Äthanol und die Propanole, an einem Dehydratisierungskatalysator zu den entsprechenden Olefinen dehydratisiert und

(d) das erhaltene Olefingemisch gegebenenfalls fraktioniert.

Der erfindungsgemäß in Stufe (a) verwendete promotierte Katalysator ist Gegenstand der Europäischen Patentanmeldung 81 100 994.3 (0 034 338).

Die zur Herstellung dieses promotierten Katalysators verwendeten Ausgangskatalysatoren enthalten 18 — 45 Gew.-%, vorzugsweise 25 — 40 Gew.-% Kupferoxid; 24 — 50 Gew.-%, vorzugsweise 30 — 45 Gew.-% Zinkoxid; 5 — 25 Gew.-%, vorzugsweise 10 — 18 Gew.-% Aluminiumoxid und 0,03 — 3,4 Gew.-%, vorzugsweise 1,7 — 2,5 Gew.-% Kalium (berechnet als $K_2O$) wobei Kupfer und Zink in einem Atomverhältnis von 0,4 — 1,9 vorliegen. Die Promotorverbindungen liegen bei diesem Katalysator in Mengen von 1 — 25 Gew.-%, vorzugsweise von 3 — 18 Gew.-% (berechnet als Oxide) vor.

Vorzugsweise werden diese promotierten Katalysatoren dadurch hergestellt, daß man entweder (a') aus einer Lösung der wasserlöslichen Salze, insbesondere der Nitrate, der Hauptbestandteile und der Promotorelemente, durch Zusatz von Alkalicarbonat, insbesondere von Kaliumcarbonat, bei 50 bis 80° C, vorzugsweise bei 60 bis 70° C, einen Niederschlag erzeugt, diesen abtrennt, wäscht und trocknet; oder (b das durch thermische Zersetzung einer Kupfer-Zink-Ammincarbonatlösung in Gegenwart von suspendierten Aluminiumoxid erhaltene Oxidgemisch mit Salzen, vorzugsweise Nitraten, der Promotorelemente imprägniert; und die nach (a') bzw. (b') erhaltenen Produkte bei 350 bis 450° C, vorzugsweise bei 380 bis 400° C, calciniert.

Besonders bevorzugte Katalysatoren werden dadurch erhalten, daß man den Niederschlag von Verfahrensvariante (a') durch kontinuierliche Fällung der wasserlöslichen Hauptbestandteile und der Promotorelemente mit Alkalicarbonat erzeugt. Hierbei führt man zweckmäßig eine wäßrige Lösung der wasserlöslichen Hauptbestandteile und der Promotorelemente und eine wäßrige Alkalicarbonatlösung einer Mischkammer mit einer Rührvorrichtung zu und überführt die Suspension des in der Mischkammer gebildeten Niederschlages zur Alterung in ein Beruhigungsgefäß. Der für die Fällung erforderliche pH-Wert, der zweckmäßig 6,8 bis 7,0 beträgt, kann leicht dadurch eingestellt werden, daß man die Zufuhrgeschwindigkeit einer der Fällungslösungen variiert.

Ferner können die mit den Promotorverbindungen imprägnierten Ausgangskatalysatoren einer Nachimprägnierung mit einer Kalumverbindung und einer Nachcalcinierung unterzogen werden, wobei der Kaliumgehalt (berechnet als $K_2O$) 0,03 bis 3,4 Gew.-%, vorzugsweise 1,7 bis 2,5 Gew.-%, beträgt.

Als Kaliumverbindungen können Kaliumhydroxid, Kaliumcarbonat, Kaliumhydrogencarbonat, Kaliumacetat, Kaliumchromat oder -dichromat, bzw. deren Gemische, verwendet werden; die Nachcalcinierung kann bei 350 bis 450° C, vorzugsweise bei 380 bis 400° C durchgeführt werden.

Anschließend werden diese promotierten Katalysatoren zur Aktivierung einer reduzierenden Nachbehandlung unterzogen, die zunächst mit Hilfe eines eine kleine Menge Wasserstoff enthaltenden inerten Gases, wie Stickstoff, durchgeführt wird. Dann wird der Wasserstoffanteil allmählich erhöht, wobei gleichzeitig die Temperatur allmählich von 170 auf 350° C erhöht wird, bis zuletzt reiner Wasserstoff zur Reduktion verwendet wird.

Die promotierten Katalysatoren nach der Europäischen Patentanm. 81 100 994.3 (0 034 338) werden zur Synthese von Methanol und höhere Alkohole enthaltenden Alkoholgemischen verwendet, die als Treibstoff bzw. als Treibstoffzusatz für Otto-Motoren verwendet werden können.

Nach der vorliegenden Erfindung dienen derartige Alkoholgemische als Rohstoffbasis zur Herstellung von Olefinen. Durch eine gezielte Promotierung mit Chrom, Mangan und Thorium sowie durch die Auswahl geeigneter Reaktionsbedingungen läßt sich die Bildung der $C_2$- und $C_3$-Alkohole steuern.

In der Stufe (a) des erfindungsgemäßen Verfahrens wird vorzugsweise ein Synthesegas, enthaltend 25 bis 30 Vol.-%, vorzugsweise 27 Vol.-% CO, 0 bis 15 Vol.-% $N_2$, 0 bis 4 Vol.-%, $CO_2$ 0 bis 7 Vol.-% $CH_4$, Rest $H_2$, bei 250 bis 400° C, vorzugsweise bei 350° C, einem Druck von 80 bis 150 bar, vorzugsweise von 100 bar, und einer Raumgeschwindigkeit von 500 bis 5000, vorzugsweise von 1500 Liter Gas pro Std. und Liter Katalysator, am promotierten Katalysator zu dem Alkoholgemisch umgesetzt.

Vorzugsweise wird die Umsetzung des Synthesegases in Stufe (a) in von Kühlmittel umflossenen Röhrenreaktoren oder in Vollraumreaktoren mit Kaltgaskühlung durchgeführt, wobei beide Reaktorsysteme gegebenenfalls mit Kreislaufführung betrieben werden.

Das in Stufe (a) erhaltene Alkoholgemisch wird in Stufe (b) unter Anwendung einer oder mehrerer Destillationskolonnen in niedrigsiedende Nebenprodukte und Methanol einerseits und höhere Alkohole und Wasser andererseits aufgetrennt. Man erhält also neben der gewünschten $C_2$- und $C_3$-Alkoholfraktion noch reines Methanol sowie ein Gemisch aus aliphatischen $C_4$- bis $C_6$-Alkoholen bei nur geringen Anteilen an Nebenprodukten.

Die Dehydratisierung der höheren aliphatischen Alkohole wird erfindungsgemäß in Stufe (c) an üblichen Dehydratisierungskatalysatoren durchgeführt, beispielsweise an $Al_2O_3$, $SiO_2$, $TiO_2$, $AlPO_4$ oder $Ca_3(PO_4)_2$. Vorzugsweise führt man die Dehydratisierung an alkalisiertem Aluminiumoxid oder Calciumphosphat durch, um Nebenreaktionen, z. B. Kondensationen und Polymerisationen der Olefine zu unterdrücken. Die Dehydratisierung wird vorzugsweise bei atmosphärischem oder erniedrigtem Druck von 0,5 bis 1 bar, vorzugsweise von 0,75 bar, bei einer Temperatur von 350 bis 500° C, vorzugsweise von 400 bis 450° C und mit einer Raumgeschwindigkeit von 1 bis 7, vorzugsweise von 2 − 4 Liter Alkoholgemisch pro Std. und Liter Katalysator durchgeführt.

Die Alkalisierung der Dehydatisierungskatalysatoren wird vorzugsweise so durchgeführt, daß beispielsweise Aluminiumoxid oder Calciumphosphat durch Tränken mit wäßrigen Lösungen der Alkalihydroxide oder Alkalicarbonaten, vorzugsweise der Hydroxide, wie LiOH, KOH, NaOH, vorzugsweise LiOH, alkalisiert werden, wobei die Konzentration an Alkalimetallen 0,1 bis 1,5 Gew.-%, vorzugsweise 0,3 bis 0,8 Gew.-%, beträgt.

Die Wahl geeigneter Reaktionsbedingungen (Temperatur 400 bis 450° C und Raumgeschwindigkeiten von 2,0 bis 2,5 kg Alkoholgemisch pro Std. und Liter Katalysator) ermöglichen Umsätze bis 91 Mol.-% und eine Selektivität von etwa 98%.

Das aus der Stufe (c) erhaltene Reaktionsgemisch wird in der Stufe (d) zunächst abgekühlt, vorzugsweise auf eine Temperatur von 10° C; anschließend wird es in flüssige und gasförmige Produkte vorgetrennt, worauf die von Wasser befreiten gasförmigen Anteile durch Tieftemperaturdestillation und die flüssigen Anteile durch Destillation oder Extraktivdestillation weiter aufgetrennt werden. Durch die Tieftemperaturdestillation können Äthylen und Propen in reiner Form gewonnen werden.

Da auch das anfallende Reinmethanol und die aliphatischen $C_4$- bis $C_6$-Alkohole verwendbar sind (beispielsweise als Lösungsmittel oder Weichmacher), wird auf diese Weise die Wirtschaftlichkeit des Gesamtverfahrens erhöht.

Nachstehend sind zunächst zwei Beispiele für die Herstellung von Dehydratisierungskatalysatoren angegeben, die in der Stufe (c) des vorliegenden Verfahrens verwendet werden können.

3

### Herstellung des Dehydratisierungskatalysators I

Aluminiumoxidhydrat-Pulver wurde mit 3,6% seines Gewichts an Aluminiumstearat vermischt und mit Hilfe einer Tablettiermaschine zu zylindrischen Tabletten mit 4,5 mm Höhe und 4,5 mm Durchmesser verpreßt. Die Tabletten wurden anschließend an Luft 4 Stunden bei 510° C calciniert.

100 g der calcinierten Tabletten wurden bei Raumtemperatur in eine wäßrige Lösung von 10 g LiOH in 435 ml Wasser 20 Minuten getaucht und danach bei 120° C 2 Stunden getrocknet und bei 400° C 2 Stunden calciniert. Das Produkt enthielt 0,29% Li und wies eine BET-Oberfläche von 210 m²/g auf.

### Herstellung des Dehydratisierungskatalysators II

Tricalciumphosphat wurde mit 3,6% seines Gewichts an Aluminiumstearat vermischt und mit Hilfe einer Tablettiermaschine zu zylindrischen Tabletten mit 4,5 mm Höhe und 4,5 mm Durchmesser verpreßt. Die Tabletten werden an anschließend an Luft 4 Stunden bei 510° C calciniert. 100 g der calcinierten Tabletten wurden bei Raumtemperatur in eine wäßrige Lösung von 10 g LiOH in 166 ml Wasser 20 Minuten getaucht und danach bei 120° C 2 Stunden getrocknet und bei 400° C 2 Stunden calciniert. Das Produkt enthielt 0,28% Li und wies eine BET-Oberfläche von 38,7 m²/g auf.

Das Verfahren gemäß der Erfindung ist anhand der nachstehenden Ausführungsbeispiele in nicht einschränkender Weise erläutert.

### Beispiel 1

In einen mit einem flüssigen Medium beheizten Rohrreaktor (Rohrdurchmesser 18 mm, Rohrlänge 1000 mm) wurden 30 ml eines zu Tabletten mit den Abmessungen 3 × 3 mm gepreßten promotierten Katalysators A eingefüllt. Der nach der Arbeitsweise von Beispiel 7 der europ. Patentanmeldung 81 100 994.3 (0 034 338) erhalten wurde, und dessen Zusammensetzung in Tabelle I angegeben ist. Der Katalysator A wurde mit einem Gas, bestehend aus 1,2 Vol.-% $H_2$, Rest $N_2$ über 40 Stunden bei 145 bis 350° C aktiviert. Der Temperaturanstieg betrug etwa 5° C/Stunde. Nach dem Erreichen von 350° C wurde der zu prüfende Katalysator noch 5 Stunden mit reinem Wasserstoff behandelt. Anschließend wurde Synthesegas mit einer Zusammensetzung von

| | |
|---|---|
| CO | 29,0 Vol.-% |
| $CO_2$ | 1,5 Vol.-% |
| $CH_4$ | 1,4 Vol.-% |
| $N_2$ | 5,0 Vol.-% |
| $H_2$ | Rest |

dem Reaktor zugeführt und ein Druck von 100 bar und eine Raumgeschwindigkeit von 2000 Liter Synthesegas pro Std. und Liter Katalysator eingestellt. Die Ergebnisse und die Zusammensetzung der Reaktionsprodukte sind in Tabelle II und III zusammengefaßt.

Die durch Kondensation bei 10° C erhaltenen flüssigen Produkte wurden destillativ so verarbeitet, daß eine Fraktion, bestehend aus Äthanol und Propanolen, gewonnen und anschließend der Dehydratisierung unterworfen wurde. Die Zusammensetzung dieser Fraktion ist in Tabelle IV angegeben.

Die Dehydratisierung des Alkoholgemisches wurde bei 400° C, einem Druck von 1 bar und einer Raumgeschwindigkeit von 2,5 kg Alkoholgemisch pro Std. und Liter Katalysator in einem elektrische beheizten Rohrreaktor mit 25 mm Rohrdurchmesser und 500 ml Rohrlänge, beschickt mit 50 ml Dehydratisierungskatalysator I durchgeführt.

Nach der Kondensation bei 10° C wurden die gasförmigen Produkte getrocknet und einer Tieftemperaturdestillation unterworfen, während die flüssigen Produkte einer Extraktivdestillation unterworfen wurden. Die Ergebnisse der Dehydratisierung und die Zusammensetzung der einzelnen Fraktionen sind in Tabelle V angegeben.

### Beispiel 2

Die Arbeitsweise von Beispiel 1 wurde mit der Abweichung wiederholt, daß zur Herstellung eines Gemisches aus Methanol und höheren aliphatischen Alkoholen der Katalysator B verwendet wurde, dessen Herstellung in Beispiel 5 der Europäischen Patentanm. 81 100 994.3 (0 034 338) beschrieben und dessen Zusammensetzung in Tabelle I angegeben ist. Die Ergebnisse und die Zusammensetzung der Reaktionsprodukte sind in Tabelle II und III angegeben. Die Zusammensetzung der durch Destillation (Stufe (b)) gewonnenen Äthanol-Propanol-Fraktion ist in Tabelle IV angegeben. Diese Fraktion wurde nach der Arbeitsweise von Beispiel 1 dehydratisiert, und das erhaltene Olefingemisch wurde fraktioniert.

## Beispiel 3

Die Arbeitsweise von Beispiel 1 wurde mit der Abweichung wiederholt, daß zur Herstellung eines Gemisches aus Methanol und höheren aliphatischen Alkoholen der Katalysator C verwendet wurde, dessen Herstellung in Beispiel 2 der Europäischen Patentanm. 81 100 994.3 (0 034 338) beschrieben und dessen Zusammensetzung in Tabelle I angegeben ist. Die Ergebnisse und die Zusammensetzung der Reaktionsprodukte sind in Tabelle II und III angegeben. Die Zusammensetzung der durch Destillation gewonnenen Äthanol-Propanol-Fraktion ist in Tabelle IV angegeben. Die Dehydratisierung und Fraktionierung wurde wie nach Beispiel 1 durchgeführt. Die Ergebnisse der Dehydratisierung und die Zusammensetzung der einzelnen Fraktionen sind in Tabelle V angegeben.

## Beispiel 4

Die Dehydratisierung einer nach der Arbeitsweise von Beispiel 1 erhaltenen Äthanol-Propanol-Fraktion wurde bei 450°C, einem Druck von 1 bar und einer Raumgeschwindigkeit von 2,0 kg Alkoholgemisch pro Std. und Liter Katalysator in einem Reaktor gemäß Beispiel 1 unter Verwendung des Dehydratisierungskatalysators II durchgeführt. Die Ergebnisse der Dehydratisierung und die Zusammensetzung der einzelnen Fraktionen sind in Tabelle V angegeben.

## Beispiel 5

Die Dehydratisierung einer nach der Arbeitsweise von Beispiel 4 erhaltenen Äthanol-Propanol-Fraktion wurde nach der Arbeitsweise von Beispiel 4 durchgeführt. Die Ergebnisse der Dehydratisierung und die Zusammensetzung der einzelnen Fraktionen sind in Tabelle V angegeben.

Tabelle I

Zusammensetzung von promotierten kupfer-, zink-, aluminium- und kaliumhaltigen Katalysatoren (Gew.-%)

| Bezeich-nung | Cu/(CuO) | ZnO | $Al_2O_3$ | K | $Cr_2O_3$ | MnO | GV[*]) | BET-Ober-fläche ($m^2$/g) |
|---|---|---|---|---|---|---|---|---|
| A | 27,3 (32,0) | 33,8 31,6 | 13,7 12,8 | 3,1 2,9 | 4,5 4,2 | 9,2 8,6 | 6,1 5,7 | 60,9 |
| B | 32,9 (38,1) | 31,7 29,3 | 15,7 14,5 | 2,9 2,7 | 8,9 8,2 | — — | 7,3 6,7 | 43,2 |
| C | 34,2 (39,4) | 32,9 30,3 | 16,4 15,1 | 3,0 2,8 | 3,3 3,0 | — — | 7,9 7,3 | 45,4 |

[*]) Glühverlust, hauptsächlich bedingt durch Kohlenstoff.

5

Tabelle II

Umsatz an CO (Mol.-%) und Selektivitäten (%) der Bildung von einzelnen Reaktionsprodukten bei 350°C, 100 bar Druck und der Raumgeschwindigkeit von 2000 Liter Synthesegas pro Std. und Liter Kat.

| Katalysator | CO-Umsatz (Mol.-%) | Selektivität (%) der Bildung von | | | |
|---|---|---|---|---|---|
| | | Methanol | Höhere aliph. Alkohole | $CO_2$ | Nebenprodukte ($CH_4$ + flüss. K. W.) |
| A | 41,3 | 42,2 | 11,8 | 44,5 | 1,5 |
| B | 42,1 | 39,5 | 18,5 | 37,8 | 4,2 |
| C | 33,8 | 45,8 | 16,2 | 36,1 | 1,9 |

Tabelle III

Ausbeute (g pro Std. und Liter Kat. und m$^3$ Synthesegas) von flüssigen Reaktionsprodukten, Methanol und höheren aliphatischen Alkoholen.

| Katalysator | Flüssige Reaktionsprodukte | Methanol | Höhere aliphatische Alkohole | | | Anteil % von Äthanol in $C_2 + C_3$ Alkoholfraktion |
|---|---|---|---|---|---|---|
| | | | Gesamt | davon $C_2 + C_3$ | $C_4 - C_6$ | |
| A | 67,3 | 48,1 | 13,1 | 10,3 | 2,8 | 48,5 |
| B | 73,7 | 47,9 | 21,9 | 11,6 | 10,3 | 36,1 |
| C | 64,4 | 39,7 | 19,4 | 11,2 | 8,2 | 25,6 |

Tabelle IV

Zusammensetzung der gewonnenen Äthanol-Propanol-Fraktion

| Alkohol | Synthese-Katalysator | | |
|---|---|---|---|
| | A | B | C |
| | Zusammensetzung der Äthanol-Propanol-Fraktion | | |
| | 1 | 2 | 3 |
| Äthanol | 46,6 | 34,4 | 24,1 |
| n-Propanol | 4,7 | 3,5 | 3,5 |
| i-Propanol | 44,7 | 57,1 | 66,3 |
| $C_4-C_6$-Alkohole | 4,0 | 5,0 | 6,1 |

Tabelle V

Die Ergebnisse der Dehydratisierung von Äthanol-Propanol-Fraktion

| Dehydra-tisierungs-katalysator | Äthanol-Propanol-Fraktion | Umsatz (Mol.-%) | Neben-produkte (Gew.-%) | Zusammensetzung der gasförmigen Produkte (Vol.-%) | | |
|---|---|---|---|---|---|---|
| | | | | $C_2H_4$ | $C_3H_6$ | höhere Olefine |
| I | 1 | 90,5 | 3,0 | 53,9 | 43,7 | 2,4 |
| I | 4 | 91,0 | 4,8 | 30,5 | 68,1 | 1,4 |
| II | 1 | 89,0 | 2,0 | 54,6 | 44,2 | 2,3 |
| II | 4 | 89,8 | 2,5 | 30,7 | 68,0 | 1,3 |

**Patentansprüche**

1. Verfahren zur Herstellung von Olefinen, insbesondere von Äthylen und Propen, durch Dehydratisierung von aliphatischen Alkoholen, dadurch gekennzeichnet, daß man

(a) aus Kohlenmonoxid und Wasserstoff enthaltenden Gasen, wie Synthesegas, unter Anwendung eines Katalysators auf der Grundlage eines Kupfer-, Zink- und gegebenenfalls Aluminiumoxid und Alkalioxid enthaltenden Ausgangskatalysators, der dadurch erhältlich ist, daß man entweder

(a') aus löslichen Salzen des Kupfers, Zinks und gegebenenfalls Aluminiums (Hauptbestandteile) durch gemeinsame Fällung mit Promotorverbindungen in alkalischem Medium einen Niederschlag erzeugt, der nach Entfernung der Fremdionen calciniert wird; oder

(b') ein Gemisch der Oxide des Kupfers, Zinks und gegebenenfalls des Aluminiums mit Lösungen der Promotorverbindungen imprägniert und das erhaltene Produkt calciniert, wobei die Promotorverbindungen Verbindungen von Chrom, Cer, Lanthan, Mangan oder von Kombinationen dieser Elemente darstellen, und wobei auf einer beliebigen Herstellungsstufe Alkaliverbindungen zugesetzt werden, ein Methanol und höhere aliphatische Alkohole enthaltendes Alkoholgemisch erzeugt;

(b) aus diesem Alkoholgemisch das Methanol abtrennt;

(c) die höhere aliphatischen Alkohole, insbesondere Äthanol und die Propanole, an einem Dehydratisierungskatalysator zu den entsprechenden Olefinen dehydratisiert und

(d) das erhaltene Olefingemisch gegebenenfalls fraktioniert.

2. Verfahren nach Anspruch 1, dadurch gekennzeichnet, daß man in der Stufe (a) einen Katalysator verwendet, der dadurch erhältlich ist, daß man entweder (a') aus einer Lösung der wasserlöslichen Salze, insbesondere der Nitrate, der Hauptbestandteile und der Promotorelemente, durch Zusatz von Alkalicarbonat, insbesondere von Kaliumcarbonat, bei 50 bis 80° C, vorzugsweise bei 60 bis 70° C, einen Niederschlag erzeugt, diesen abtrennt, wäscht und trocknet; oder (b') das durch thermische Zersetzung einer Kupfer-Zink-Ammincarbonatlösung in Gegenwart von suspendierten Aluminiumoxid erhaltene Oxidgemisch mit Salzen, vorzugsweise Nitraten, der Promotorelemente imprägniert; und die nach (a') bzw. (b') erhaltenen Produkte bei 350 bis 450° C, vorzugsweise bei 380 bis 400° C calciniert.

3. Verfahren nach einem der Ansprüche 1 oder 2, dadurch gekennzeichnet, daß man in der Stufe (a) einen Katalysator verwendet, der dadurch erhältlich ist, daß man den Niederschlag von Herstellungsvariante (a') durch kontinuierliche Fällung der wasserlöslichen Hauptbestandteile und der Promotorelemente mit Alkalicarbonat erzeugt.

4. Verfahren nach einem der Ansprüche 1 bis 3, dadurch gekennzeichnet, daß man in der Stufe (a) einen Katalysator verwendet, der dadurch erhältlich ist, daß man eine wäßrige Lösung der wasserlöslichen Hauptbestandteile- und der Promotorelemente und eine wäßrige Alkalicarbonatlösung einer Mischkammer mit einer Rührvorrichtung zuführt und die Suspension des in der Mischkammer gebildeten Niederschlages zur Alterung in ein Beruhigungsgefäß überführt.

5. Verfahren nach einem der Ansprüche 1–4, dadurch gekennzeichnet, daß man in der Stufe (a) ein Synthesegas, enthaltend 25 bis 30 Vol.-%, vorzugsweise 27 Vol.-% CO, 0 bis 15 Vol.-% $N_2$, 0 bis 4 Vol.-% $CO_2$, 0–7 Vol.-% $CH_4$, Rest $H_2$, bei 250 bis 400° C, vorzugsweise bei 350° C, einem Druck von 80 bis 150 bar, vorzugsweise von 100 bar, und einer Raumgeschwindigkeit von 500 bis 5000, vorzugsweise von 1500 Liter Gas pro Std. und Liter Katalysator, am promotierten Katalysator zu dem Alkoholgemisch

umsetzt.

6. Verfahren nach einem der Ansprüche 1 — 5, dadurch gekennzeichnet, daß man die Umsetzung des Synthesegases in Stufe (a) in vom Kühlmittel umflossenen Röhrenreaktoren oder in Vollraumreaktoren mit Kaltglaskühlung durchführt, wobei beide Reaktorsysteme gegebenenfalls mit Kreislaufführung betrieben werden.

7. Verfahren nach einem der Ansprüche 1 bis 6, dadurch gekennzeichnet, daß man das in Stufe (a) erhaltene Alkoholgemisch in Stufe (b) unter Anwendung einer oder mehrerer Destillationskolonnen in niedrigsiedende Nebenprodukte und Methanol einerseits und in höhere Alkohole und Wasser andererseits auftrennt.

8. Verfahren nach einem der Ansprüche 1 bis 7, dadurch gekennzeichnet, daß man die höheren aliphatischen Alkohole in Stufe (c) an alkalisiertem Aluminiumoxid oder Calciumphosphat dehydratisiert.

9. Verfahren nach Anspruch 8, dadurch gekennzeichnet, daß man die Dehydratisierung in Stufe (c) bei atmosphärischem oder erniedrigtem Druck von 0,5 bis 1 bar, vorzugsweise von 0,75 bar einer Temperatur von 350 bis 500° C, vorzugsweise von 400 bis 450° C, und mit einer Raumgeschwindigkeit von 1 bis 7, vorzugsweise von 2 bis 4 Liter Alkoholgemisch pro Std. und Liter Katalysator, durchführt.

10. Verfahren nach einem der Ansprüche 1 bis 9, dadurch gekennzeichnet, daß man die Dehydratisierung in Stufe (c) an Aluminiumoxid oder Calciumphosphat durchführt, die durch Tränken mit wäßrigen Lösungen der Alkalihydroxide oder Alkalicarbonate, vorzugsweise der Hydroxide, wie LiOH, KOH, NaOH, vorzugsweise LiOH, alkalisiert worden sind, wobei die Konzentration an Alkalimetallen 0,1 bis 1,5 Gew.-%, vorzugsweise 0,3 bis 0,8 Gew.-% beträgt.

11. Verfahren nach einem der Ansprüche 1 bis 10, dadurch gekennzeichnet, daß man das aus der Stufe (c) erhaltene Reaktionsgemisch in der Stufe (d) zunächst bis auf eine Temperatur von 10° C abkühlt und in flüssige und gasförmige Produkte vortrennt, worauf man die von Wasser befreiten gasförmigen Anteile durch Tieftemperaturdestillation und die flüssigen Anteile durch eine Destillation oder Extraktivdestillation weiter auftrennt.

## Claims

1. Process for the production of olefins, in particular of ethylene and propene by dehydration of aliphatic alcohols, characterised in that an alcohol mixture containing methanol and higher aliphatic alcohols is produced

(a) from gases containing carbon dioxide and hydrogen, such as synthesis gas, using a catalyst based on a starting catalyst containing copper oxide, zinc oxide and, optionally, aluminium oxide and alkali oxide, which starting catalyst starting catalyst is obtainable either,

(a') by producing form soluble salts of copper, zinc and, optionally, aluminium (principal constituents) a precipitate by joint precipitation with promoter compounds in alkaline medium, which precipitate is calcined after removal of the foreign ions; or

(b') by impregnating a mixture of the oxides of copper, zinc and, optionally, of aluminium with solutions of the promoter compounds and by calcining the product obtained, in which case the promoter compounds constitute compounds of chromium, cerium, lanthanum, manganese or combinations of these elements, and in which case alkli compounds are added at any production stage desired;

(b) the methanol is separated from this alcohol mixture;

(c) the higher aliphatic alcohols, in particular ethanol and the propanols, are dehydrated on an dehydrating catalyst to form the corresponding olefins and

(d) the olefin mixture obtained is optionally fractionated.

2. Process according to Claim 1, characterised in that in step (a) a catalyst is used which is obtainable either (a') by producing a precipitate from a solution of the water-soluble salts, in particular the nitrates, the principal constituents and the promoter elements, by addition of alkali carbonate, in particular of potassium carbonate, at 50 to 80° C, preferably at 60 to 70° C, which precipitate is separated, washed and dried; or (b') by impregnating the oxide mixture obtained by thermal decomposition of a copper-zinc-ammine carbinate solution in the presence of suspended aluminium oxide with salts, preferably nitrates, of the promoter elements; and the products obtained respectively according to (a') or (b') are calcined at 350 to 450° C, preferably at 380 to 400° C.

3. Process according to one of Claims 1 or 2, characterised in that in step (a) a catalyst is used which is obtainable by producing the precipitate of production variant (a') by continuous precipitation of the water-soluble principal constituents and of the promoter elements with alkali carbonate.

4. Process according to one of Claims 1 to 3, characterised in that in step (a) a catalyst is used which is obtainable by feeding an aqueous solution of the water-soluble principal constituents and of the promoter elements and an aqueous carbonate solution to a mixing chamber with an agitating device and transferring the suspension of the precipitate formed in the mixing chamber to a stabilising vessel for ageing.

5. Process according to one of Claims 1–4, characterised in that in step (a) a synthesis gas containing 25 to 30% by vol., preferably 27% by vol. of CO, 0 to 15% by vol. of $N_2$, 0 to 4% by vol. of $CO_2$, 0–7% by vol. of $CH_4$, the remainder $H_2$, at 250 to 400° C, preferably at 350° C, at a pressure of from 80 to 150 bars, preferably of 100 bars, and at a spatial velocity of from 500 to 5000, preferably of 1500 litres of gas per hour and litres of catalyst, is reacted on the promoted catalyst to form the alcohol mixture.

6. Process according to one of Claims 1 to 5, characterised in that the reaction of the synthesis gas in step (a) is carried out in tubular reactors around which coolant flows or in full volume reactors with cold gas cooling, in which case both reactor systems are operated optionally with circulation guidance.

7. Process according to one of Claims 1 to 6, characterised in that the alcohol mixture obtained in step (a) is separated in step (b) using one or more distillation columns, into low-boiling by-products and methanol, on the one hand, and into higher alcohols and water, on the other hand.

8. Process according to one of Claims 1 to 7, characterised in that the step (c) the higher aliphatic alcohols are dehydrated on alkalised aluminium oxide or calcium phosphate.

9. Process according to Claim 8, characterised in that the dehydration in step (c) is carried out at atmospheric or reduced pressure of from 0.5 to 1 bar, preferably of 0.75 bar, at a temperature of from 350 to 500° C, preferably of from 400 to 450° C, and at a spatial velocity of from 1 to 7, preferably of from 2 to 4, litres of alcohol mixture per hour and litres of catalyst.

10, Process according to one of Claims 1 to 9, characterised in that the dehydration in step (c) is carried out on aluminium oxide or calcium phosphate which has been alkalised by impregnation with aqueous solutions of alkali hydroxides of alkali carbonates, preferably hydroxides, such as LiOH, KOH, NaOH, prefereably LiOH, the concentration of alkali metals amounting to 0.1 to 1.5% by weight, preferably 0.3 to 0.8% by weight.

11. Process according to one of Claims 1 to 10, characterised in that the reaction mixture obtained from step (c) is first cooled in step (d) down to a temperature of 10° C and is pre-separated into liquid and gaseous products, whereupon the gaseous fractions freed of water are further separated by low-temperature distillation and the liquid fractions are further separated by distillation or extractive distillation.

**Revendications**

1. Procédé de préparation d'oléfines, notamment d'éthylène et de propène, par déshydratation d'alcools aliphatiques, caractérisé en ce qu'on produit

(a) à partir de monoxyde de carbone et de gaz hydrogénés, comme du gaz synthétique, en utilisant un catalyseur à base d'un catalyseur initial renfermant de l'oxyde de cuivre, de l'oxyde de zinc et éventuellement de l'alumine, que l'on peut obtenir, ou bien

   (a') en produisant à partir de sels solubles de cuivre, de zinc et éventuellement d'aluminium (composants principaux) par précipitation commune avec des composés promoteurs en milieu alcalin un précipité que l'on calcine après élimination des ions étrangers, ou bien

   (b') en imprégnant un mélange d'oxydes de cuivre, de zinc et éventuellement d'aluminium de solutions des composés promoteurs et en calcinant le produit obtenue, les composés promoteurs étant représentés par des composés de chrome, de cérium, de lanthane, de manganèse ou de combinaisons de ces éléments, et composés alcalins étant ajoutés à un stade de préparation quelconque, en produisant un mélange d'alcools renfermant du méthanol et des alcools aliphatiques supérieurs;

(b) on sépare le méthanol de ce mélange d'alcools;

(c) on déshydrate les alcools aliphatiques supérieurs, notamment l'éthanol et les propanols, sur un catalyseur de déshydratation, en les oléfines correspondantes, et

(d) on fractionne éventuellement le mélange d'oléfines obtenu.

2. Procédé selon la revendication 1, caractérisé en ce qu'on utilise, au cours de l'étape (a), un catalyseur que l'on peut obtenir, ou bien (a') en produisant à partir d'une solution des sels solubles dans l'eau, notamment des nitrates, des composants principaux et des éléments promoteurs, en ajoutant du carbonate alcalin, notamment du carbonate de potassium, à 50 à 80° C, notamment à 60 à 70° C, un précipité, en le séparant, en le lavant et en le séchant; ou bien (b') en imprégnant le mélange d'oxydes obtenus par décomposition thermique d'une solution cuivre-zinc, carbonate ammoniacal, en présence d'alumine en suspension, de sels, en particulier de nitrates, des éléments d'activation; et on calcine les produits obtenus selon (a') ou (b') à 350 à 450° C, de préférence à 380 à 400° C.

3. Procédé selon la revendivation 1 ou 2, caractérisé en ce qu'on utilise au cours de l'étape (a) un catalyseur que l'on peut obtenir en produisant le précipité de la variante de préparation (a') par précipitation continue des composants principaux solubles dans l'eau et des éléments promoteurs par du carbonate alcalin.

4. Procédé selon l'une quelconque des revendications 1 à 3, caractérisé en ce qu'on utilise au cours de l'étape (a) un catalyseur que l'on peut obtenir en envoyant une solution aqueuse des composants solubles dans l'eau et des éléments promoteurs et une solution de carbonate alcalin dans une chambre de mélange pourvue d'un agitateur, et en faisant passer la suspension due précipité formé dans la chambre de mélange dans un récipient de stabilisation, aux fins de viellissement.

5. Procédé selon l'une des revendications 1 à 4, en ce que l'on transforme au cours de l'étape (a) un gaz synthétique renfermant 25 à 30% en volume, de préférence 27% de CO, 0 à 15% en volume de $N_2$, 0 à 15% en volume de $N_2$, 0 á 4% en volume de $CO_2$, 0 à 7% en volume de $CH_4$, le reste d'$H_2$, à une température de 250 à 400°C, de préférence de 350°C, sous une pression de 80 à 150 bars, et avec un débit de 500 à 5000, de préférence 1500 litres de gaz par heure et litre de catalyseur, sur le catalyseur activé, en le mélange d'alcools.

6. Procédé selon l'une des revendications 1 à 5, caractérisé en ce que l'on effectue la transformation du gaz de synthèse au cours de l'étape (a) dans des réacteurs tubulaires entourés d'agent de réfrigération, ou dans des réacteurs à volume complet à refroidissement par gaz froids, les deux systèmes de réacteurs fonctionnant éventuellement avec recyclage.

7. Procédé selon l'une des revendications 1 à 6, caractérisé en ce que l'on sépare le mélange d'alcools obtenu au cours de l'étape (a), lors de l'étape (b) en utilisant une ou plusieurs colonnes de distillation, en sous-produits à point d'ébullition bas et en méthanol d'une part, et en alcools supérieurs et en eau d'autre part.

8. Procédé selon l'une des revendications 1 à 7, caractérisé en ce que l'on déshydrate les alcools aliphatiques supérieurs au cours de l'étape (c), sur de l'alumine alcalinisée ou du phosphate de calcium.

9. Procédé selon la revendication 8, caractérisé en ce que l'on effectue la déshydratation, au cours de l'étape (c), à la pression atmosphérique ou une pression inférieure de 0,5 à 1 bar, de préférence de 0,85 bar, une température de 350 à 500°C, de préférence de 400 à 450°C, et avec un débit de 1 à 7, de préférence 2 à 4 litres de mélange d'alcools par heure et litre de catalyseur.

10. Procédé selon l'une des revendications 1 à 9, caractérisé en ce que l'on effectue la déshydratation de l'étape (c) sur de l'alumine ou du phosphate de calcium, que l'on a alcalinisé par imprégnation des solutions aqueuses d'hydroxydes alcalins ou de carbonates alcalins, de préférence d'hydroxydes tels que LiOH, KOH, NaOH, de préférence LiOH la concentration en métaux alcalins étant de 0,1 à 1,5% en poids, de préférence de 0,3 à 0,8% en poids.

11. Procédé selon l'une des revendications 1 à 10, caractérisé en ce que l'on refroidit le mélange réactionnel obtenu au cours de l'étape (c), à l'étape (d), à une température de 10°C et on le présépare en produits liquides et gazeux, puis l'on sépare encore les fractions gazeuses débarrassées d'eau par distillation à basse température et les fractions liquides par distillation ou distillation extractive.